(19) Europäisches Patentamt
European Patent Office
Office européen des brevets

(11) Veröffentlichungsnummer: **0 512 319 A2**

# (12) EUROPÄISCHE PATENTANMELDUNG

(21) Anmeldenummer: **92106847.4**

(22) Anmeldetag: **22.04.92**

(51) Int. Cl.[5]: **C08B 5/00**, C08B 13/00, C08B 15/06, //C08B31/00, C08B37/00

(30) Priorität: **04.05.91 DE 4114588**

(43) Veröffentlichungstag der Anmeldung: **11.11.92 Patentblatt 92/46**

(84) Benannte Vertragsstaaten: **BE DE FR GB IT NL SE**

(71) Anmelder: **Wolff Walsrode Aktiengesellschaft**
**Postfach**
**W-3030 Walsrode 1(DE)**

(72) Erfinder: **Buysch, Hans-Josef, Dr.**
**Brandenburger Strasse 28**
**W-4150 Krefeld(DE)**
Erfinder: **Szablikowski, Klaus, Dr.**
**Claudiusstrasse 5**
**W-3030 Walsrode(DE)**
Erfinder: **Breckwoldt, Jörn, Dr.**
**Rostocker Strasse 55**
**W-2720 Rotenburg(DE)**

(74) Vertreter: **Braun, Rolf, Dr.**
**c/o Bayer AG Konzernverwaltung RP Patente Konzern**
**W-5090 Leverkusen 1 Bayerwerk(DE)**

## (54) Verfahren zur Herstellung von Kohlensäurederivaten von Polysacchariden.

(57) Verfahren zur Herstellung von Kohlensäurederivaten von Polysacchariden durch Umsetzung mit reaktiven Kohlensäurederivaten, dadurch gekennzeichnet, daß man Polysaccharide zunächst mit Phosgen und gegebenenfalls weiter mit protischen nucleophilen Substanzen und/oder deren Salzen der allgemeinen Formel (I)

R-X-M (I)

umsetzt, worin

R $C_{1-18}$-Alkyl, $C_{6-12}$-Aryl, $C_{7-12}$-Aralkyl, jeweils substituiert mit Olefin-, Alkin-, Amino-, Ether-, Thioether-, Carboxy-, Carbalkoxy-, Urethan-, quartären Ammoniumgruppen oder Halogenatomen,

X O oder S, gegebenenfalls als Anion oder $NR^1$ mit $R^1$ H, $C_{1-6}$-Alkyl, Phenyl, $C_{7-8}$-Aralkyl und

M H, ein Alkalikation oder ein Ammoniumion, wenn X = $O^{\ominus}$ oder $S^{\ominus}$ darstellt.

EP 0 512 319 A2

Die vorliegende Erfindung betrifft ein Verfahren zur Herstellung von Kohlensäurederivaten von Polysacchariden durch Umsetzung der Polysaccharide mit Phosgen und gegebenenfalls weiter mit protischen nucleophilen Substanzen und/oder deren Salzen der allgemeinen Formel (I)

R-X-M (I)

worin

R $C_{1-18}$-Alkyl, $C_{6-12}$-Aryl, $C_{7-12}$-Aralkyl, jeweils substituiert mit Olefin-, Alkin-, Amino-, Ether-, Thioether-, Carboxy-, Carbalkoxy-, Urethan-, quartären Ammoniumgruppen oder Halogenatomen,

x O oder S, gegebenenfalls als Anion oder $NR^1$ mit $R^1$ H, $C_{1-6}$-Alkyl, Phenyl, $C_{7-8}$-Aralkyl und

M H, ein Alkalikation oder ein Ammoniumion, wenn X = $O^{\ominus}$ oder $S^{\ominus}$ darstellt.

Kohlensäureester von Polysacchariden sind interessante Ausgangsprodukte zur Herstellung weiterer modifizierter Polysaccharide, besonders von Carbamaten mit unterschiedlichen Eigenschaften. Eine besonders aktuelle Verwendung finden sie zur Herstellung von Depots für bioaktive Materialien [vergl. Makromol. Chem. 186, 17-29 (1985)] und zur Fixierung von Enzymen für die Durchführung enzymatischer Reaktionen in heterogener Phase (vergl. z.B. J.C.S. Perkin I 1974, 757-762 oder Biochemistry Internat. 4 (1982) 629-635). Darum hat es nicht an Bemühungen gefehlt, Kohlensäureester verschiedener Polysaccharide herzustellen. Für die genannten Anwendungen, besonders die Fixierung von Enzymen ist ein hoher DS wünschenswert, damit eine hohe Enzymdichte am Polysaccharid, hohe Aktivität und geringe Katalysatorvolumina für die enzymatischen Reaktionen gewährleistet sind.

Der überwiegende Teil der bisherigen Verfahren arbeitet in homogener Phase, also mit gelöstem Polysaccharid, wobei in der Regel nur niedrig substituierte Polysaccharide erhalten werden. Darüber hinaus bringt die Umsetzung in homogener Phase wegen der häufig sehr hohen Viskosität solcher Lösungen und der daher notwendigen Verwendung riesiger Mengen an Lösungsmitteln bei der Reaktion und Ausfällung der umgesetzten Polymeren große Probleme mit sich.

Nach E. Heuser und F. Schneider, Ber. deut. chem. Ges. 57, 1389-1392 (1924) soll man durch homogene Reaktion von aus Viskose gewonnener Hydratcellulose in 8 %iger Natronlauge eine Methylcarbonatcellulose mit einem DS von maximal 2 erhalten, der aber wegen der Anwesenheit der verseifenden Natronlauge ein Zufallsergebnis und nicht reproduzierbar ist. Die Ausbeuten liegen immer unter 20 %. Damit ist dieses Verfahren als völlig unbrauchbar einzustufen.

In Lösungen von Dimethylsulfoxid lassen sich Stärke, Dextrin und Dextran (Carbohydrate Research 8 (1968), 266-274) bis zu einem maximalen DS von 0,4 in Carbonat-Polysaccharide überführen. Heterogene Umsetzungen von Cellulose in Dimethylsulfoxid oder Dimethylformamid führen nach Carbohydrate Research 17 (1971), 471-4 aufgrund spektroskopischer Analysen zu einem DS um 0,5. Der nach Umsetzung des Carbonats mit $NH_3$ zum Carbamat zu ermittelnde Stickstoffwert von 0,8 läßt allerdings einen wesentlich geringeren DS von <0,2 vermuten.

In J.C.S. Perkin I 1973, 2293-2299 wird berichtet, daß makroporöse Cellulose ebenfalls in einer Suspension in Dimethylsulfoxid nach Umsetzung mit Chlorkohlensäureethylester zu einem Cellulosecarbonat von DS <0,5 führt (errechnet aus der maximalen $NH_3$-Aufnahme 3 mmol/g Matrix).

Dextrane lassen sich in einer Lösung von Dimethylformamid, die LiCl enthält, mit Chlorkohlensäureethyl- oder -butylester in Gegenwart verschiedener Basen hoch substituieren bis zu einem DS von 3,0, s. Makromol. Chemie (1985) 17-29, der allerdings aus spektroskopischen Daten errechnet wurde. Die Angaben des Carbonatgehaltes in Mol-% lassen allerdings auf wesentlich geringere Substitutionsgrade im Bereich von maximal 1,5 schließen. In dieser Publikation wird darauf hingewiesen, daß die Reaktionsgeschwindigkeit und der Substitutionsgrad von der Basizität der verwendeten Hilfsbase abhängt. Demnach werden die besten Ergebnisse mit Triethylamin erhalten. Trotz der hohen Substitutionsgrade ist dieses Verfahren aus den o.g. Gründen wegen des komplexen Lösungsmittelgemisches und der damit verbundenen schwierigen Aufarbeitung für technische Zwecke ungeeignet. Außerdem erfolgt unter den Reaktionsbedingungen eine Zersetzung der Acylhalogenide.

Verwendet man aromatische Chlorkohlensäureester, z.B. den p-Nitrophenylester, und arbeitet in Dimethylsulfoxid als Lösungsmittel für Dextran, so findet man einen wesentlich geringeren DS von ca. 0,3, wie sich aus der prozentualen Aktivierung von max. 30 % (Anzahl der Carbonatgruppen pro 100 Anhydroglukoseeinheiten) errechnet. Außerdem zeigt sich, daS die Anzahl der Carbonatgruppen während der Reaktion durch ein Maximum verläuft, also die Carbonatgruppe unter den Reaktionsbedingungen nicht beständig ist (Makromol. Chem. 186, 2455 bis 2460 (1985)).

Ähnliches gilt für Umsetzungen mit aromatischen Chlorkohlensäureestern an gelförmiger Sepharose, die in trockenem Pyridin/Aceton-Gemisch suspendiert wird (Biochemistry International 4 (1982), 629-635). Man erhält nur einen geringen DS bis zu 0,28 (errechnet aus der Angabe von max. 1,8 mmol aktiven

Gruppen pro g Polymer).

Nach EP-A 367 002 erhält man Kohlensäurederivate von Polysacchariden, wenn man Polysaccharide mit Chlorkohlensäureestern in Gegenwart einer Base in heterogener Phase zu Carbonaten und diese gegebenenfalls mit Aminen zu Urethanen umsetzt.

Dieses Verfahren hat zwar den Vorteil, daß man hohe Substitutionsgrade erzielt, jedoch auch den Nachteil, daß man zunächst aus Phosgen und Hydroxyverbindungen die entsprechenden Chlorkohlensäureester herstellen, diese mit Polysacchariden zu Carbonaten und weiter mit Aminen zu Urethanen verarbeiten muß, wie beispielsweise in der EP-A 367 003 beschrieben. Die dabei aus den Polysaccharidcarbonaten wiederum abgespaltenen Hydroxyverbindungen müssen von den Polysacchariden abgetrennt und wiedergewonnen werden.

Das vorliegende Verfahren bringt demgegenüber eine erhebliche Vereinfachung der Synthese von Polysaccharid-Carbonsäurederivaten, da man direkt von Phosgen ausgehen und ohne Bildung weiterer Koppelprodukte als Chlorid auch zu komplexeren Derivaten wie Arylcarbonaten, Thiocarbonaten oder Urethanen vorstoßen kann. Auch nach vereinfachten erfindungsgemäßen Verfahren sind hohe Substitutionsgrade zu erzielen.

Aufgabe der Erfindung war es also, ein einfaches Verfahren zur Herstellung von Kohlensäurederivaten von Polysacchariden mit hohen Substitutionsgraden zu entwickeln.

Gegenstand der Erfindung ist daher ein Verfahren zur Herstellung von Polysaccharid-Kohlensäurederivaten, gekennzeichnet durch Umsetzen von Polysacchariden mit Phosgen und gegebenenfalls weiter mit protischen Nucleophilen oder deren Salzen der allgemeinen Formel (I).

Geeignete Ausgangsmaterialien zur Herstellung der erfindungsgemäßen Polysaccharidcarbonate sind beispielsweise Polyglucosane wie Cellulose, die verschiedenen Derivate der Cellulose wie Methylcellulose, Hydroxyethylcellulose, Hydroxypropylcellulose oder gemischte Celluloseether wie Methyl-hydroxyethylcellulosen, Methylhydroxypropylcellulose, Sulfoethylcellulose, Sulfoethylcarboxymethylcellulose, Methylsulfoethylcellulose, Hydroxyethylsulfoethylcellulose, Dihydroxypropylcellulose, Dihydroxypropylhydroxyethylcellulose, Dihydroxypropylcarboxymethylcellose, Carboxymethylcellulose, ihre verschiedenen Salze mit Natrium-, Kalium-, Calcium- oder Ammonium-besonders quartären Ammoniumionen; Cellulosesulfat mit verschiedenen Gegenionen, etwa des Natriums, Kaliums, Calciums, Ammoniums und quartärer Ammoniumgruppen;
Stärke, Dextrine, Glycogen;
Polyfructosane wie Inulin und Graminin;
Polymannosane, Polygalactosane;
auch gemischte Polysaccharide wie Hemicellulosen ferner Polyxylosane und Polyarabinosane, sowie auch Heteropolysaccharide wie Gellan, Xanthan und Pullulan.

Bevorzugte Ausgangsprodukte sind Cellulose und ihre Derivate, Stärke und Dextrine, besonders bevorzugt sind Cellulose, Methylcellulose, Ethylcellulose, Hydroxyethylcellulose, Hydroxypropylcellulose, Carboxymethylcellulose und deren Salze und Stärke.

Das Verfahren kann in Lösung durchgeführt werden, Bevorzugt jedoch wird eine Umsetzung der Polysaccharide in suspendierter oder dispergierter Form, also in heterogener Phase, da auf diese weise das Arbeiten in hochviskosen Medien, Fällen der umgesetzten Polysaccharide aus der Lösung und die Aufarbeitung riesiger Lösungsmittelmengen vermieden wird.

Geeignete Dispersionsmedien sind unter den Reaktionsbedingungen inerte Lösungsmittel wie aliphatische und aromatische Kohlenwasserstoffe, Halogenkohlenwasserstoffe, Ether, Ester, Ketone, Carbonsäureamide und Carbonsäurenitrile.

Genannt seien beispielsweise Cyclohexan, Pentan, Heptan, Isooctan, Benzol, Toluol, Methylenchlorid, Chloroform, Dichlorethan, Trichlorethylen, Chlorbenzol, Dichlorbenzol, Brombenzol, Diethylether, Diisopropylether, Dibutylether, Dioxan, Benzodioxan, Anisol, Dimethoxybenzol, Ethylenglykoldimethylether, Diethylenglykol-dimethylether, Essigsäureethylester, Essigsäurebutylester, Propionsäureethylester, Buttersäureethylester, Benzoesäureethylester, Malonsäurediethylester, Bernsteinsäurediethylester, Aceton, Methylethylketon, Diethylketon, Methylisopropylketon, Acetophenon, Cyclohexanon, Formamid, Methylformamid, Dimethylformamid, Dimethylacetamid, Tetramethylharnstoff, N,N-Dimethyl-ethylenharnstoff, N,N-Dimethyl-2,6-diaza-4-oxa-cyclohexanon, N-Methylpyrrolidon, N-Methylcaprolactam, Acetonitril, Propionitril, Butyronitril, Benzonitril, Adiponitril, $\beta$-Methoxy-propionitril und $\beta$-Cyano-$\beta$'-methoxy-diethylether.

Die Dispersionsmediummenge soll so bemessen sein, daß sich eine gut rührbare Suspension bildet.

Die Reaktion der Polysaccharide mit Phosgen kann in Gegenwart oder in Abwesenheit von Basen durchgeführt werden. Geeignete Basen für die Umsetzung sind tertiäre Stickstoffbasen aus der aliphatischen, aromatischen und heterocyclischen Reihe wie Trimethylamin, Triethylamin, Tributylamin, Dimethylcyclohexylamin, Diisopropylethylamin, Dicyclohexyl-methylamin, Dimethyl-$\beta$-methoxyethylamin, N,N,N',N'-Tetramethylethylendiamin, N,N,N',N'-Tetramethyl-$\beta$,$\beta$'-diamino-diethylether, N,N'-Dimethylpiperazin, N-Methyl-morpholin und Diazabicyclooctan, N,N-Dimethylanilin, N,N-Diethylanilin, N,N,N',N'-Tetramethyldiaminobenzol, N,N-Dimethyl-toluidin, N,N-Diethyl-xylidin, N,N-Dimethylanisidin und N-Phenylmorpholin, Pyrazol, N-Alkylpyrazol, Imidazol, N-Methylimidazol, Triazol, N-Ethyltriazol, N,N-Dimethylamino-imidazol, N,N-Diethylamino-triazol, Pyridin, $\alpha$, $\beta$-, $\gamma$-Picolin, die Lutidine, Collidin, Ethyl-methylpyridine, N,N-Dialkylaminopyridine wie N,N-Dimethyl-4-aminopyridin, Chinolin, Methylchinolin und Isochinolin.

Bevorzugt sind Triethylamin, Dimethylcyclohexylamin, Methylmorpholin, Dimethylpiperazin und Diazabicyclooctan, Tetramethylethylendiamin, N,N-Dimethylanilin und N,N-Dimethylanisidin, Imidazol, N-Methylimidazol, N,N-Dimethylamino-imidazol,Pyridin, Chinolin und die Methyl- und Ethylpyridine, besonders bevorzugt aber sind Pyridin und die Methylpyridine.

Das Molverhältnis von Base zu Phosgen sollte mindestens 1 : 1 betragen. Es ist jedoch auch möglich, mehr als 1 Mol Base pro Mol Phosgen einzusetzen. Dadurch wird der Anteil an Chlorkohlensäureester im Polysaccharid-Kohlensäurederivat reduziert und der Anteil an Carbonat erhöht. Es ist sogar möglich, die Umsetzung ausschließlich in einer Base durchzuführen, wobei die Base gleichzeitig als Dispersionsmittel dient.

Ausgangsmaterialien, Reagentien, Basen und Solventien sollten im wesentlichen wasserfrei sein. Deshalb ist es erforderlich, die Reaktionspartner und Dispersionsmedien von überschüssigem Wasser zu befreien oder eine entsprechend größere Menge an Phosgen zu verwenden, um damit überschüssiges Wasser zu kompensieren. Dies ist jedoch ein wenig ökonomisches Verfahren. Vorteilhafter ist eine Trocknung der gesamten Einsatzmaterialien mit üblichen Mitteln und Methoden, beispielsweise bei erhöhter Temperatur im Vakuum, durch azeotrope Destillation, durch Trocknen über Alkalihydroxiden wie NaOH oder KOH oder Erdalkalihydroxiden oder -oxiden, wie $Ca(OH)_2$ oder CaO, über entwässerten Salzen wie Natriumsulfat oder Kupfersulfat oder über Molekularsieben, die sich leicht regenerieren lassen.

Wird dabei das Wasser soweit entzogen, daß die Polysaccharide nur noch das nicht entfernbare gebundene Wasser enthalten, so ist die Reproduzierbarkeit der Umsetzung gefährdet und man setzt deshalb mindestens 0,5 Gew.-% bezogen auf das umzusetzende Polysaccharid zu.

Der Wasserzusatz ist nach oben durch ökonomische Überlegungen begrenzt, da ein Teil des Acylierungsmittels verloren geht, deshalb gibt man in der Regel 0,5 bis 5 Gew.-%, bevorzugt 0,8 bis 4,0 Gew.-%, besonders bevorzugt 1,0 bis 3,0 Gew.-% hinzu. Der Kristallwasser-Gehalt von Polysacchariden ist unterschiedlich und kann durch geeignete Analysenmethoden bestimmt werden (vgl. "Starch Chemistry and Technology" ed. by R.L. Whistler, J.N. Bemiller, E.F. Paschall 1983 sec. Ed. S. 214 bis 219, Academic Press Inc. N.Y.).

Der Feuchtegehalt von Cellulose wird nach der Analysenmethode vom Verein der Zellstoff- und Papier-Chemiker und -Ingenieure, Merkblatt IV/42/67 vom 23.10.1967 bestimmt.

Für Cellulose beträgt er beispielsweise je nach Typ und Herkommen zwischen 2 und 4 Gew.-%. Angaben über gebundenes Wasser oder Feuchtegehalt können der einschlägigen Literatur entnommen werden (vgl. K. Götze: Chemiefasern, Bd. 1, S. 264, 3. Aufl. 1964 Springer Verlag).

Da das Phosgen wasserfrei ist, wird die fehlende Wassermenge im allgemeinen der Polysaccharidsuspension vor der Dosierung des Phosgens beigemischt. Die Reaktion wird zweckmäßigerweise so durchgeführt, daß man das Polysaccharid mit der Base, gegebenenfalls zusätzlichem Wasser und gegebenenfalls dem Dispersionsmedium, das das Polysaccharid nicht löst, vorlegt, je nach Art des Polysaccharids gegebenenfalls eine Vorbehandlung anschließt und dann das Phosgen zudosiert.

Eine Vorbehandlung erfolgt zweckmäßig dann, wenn das Polysaccharid, wie im Falle der Cellulose für die Umsetzung aufgeschlossen werden soll. Die Temperatur kann zwischen 10 und 120 °C variieren und ist nicht kritisch. Die Zeit für die Vorbehandlung ist ebenfalls nicht kritisch. Sie kann zwischen mehreren Minuten und vielen Stunden liegen. In der Regel liegt sie zwischen 1 bis 30 h. Eine solche Vorbehandlung ist jedoch nicht zwingend vorgeschrieben, da der notwendige Aufschluß auch während der Umsetzung erfolgen kann.

Man kann auch mit wäßrigem Alkalihydroxid vorbehandelte, anschließend von Alkalihydroxid und überschüssigem Wasser befreite Polysaccharide in die Reaktion einsetzn. Dies kann besonders bei Cellulosen in einzelnen Fällen vorteilhaft sein und aufgrund eines besseren Aufschlusses den Substitutionsgrad noch etwas erhöhen. Dafür eignen sich besondere 8 bis 25 gew.-%ige Natronlaugen, die anschließend mit Wasser ausgewaschen werden und das Wasser mit Alkoholen wie Isopropanol oder der Hilfsbase. Der Alkohol kann durch Trocknen oder Verdrängen mit dem Dispersionsmedium für die Umsetzung entfernt werden.

Die Menge an Phosgen, die mit den Polysacchariden zur Reaktion gebracht werden soll, hängt ab von der Vorsubstitution des Polysaccharids und von dem angestrebten Substitutionsgrad. Sie liegt demnach zwischen etwa 0,05 bis 9,0 Mol pro Molgewichtseinheit des Polysaccharides, bevorzugt bei 0,1 bis 6,0 Mol.

Wenn beispielsweise eine Methylcellulose mit einem Substitutionsgrad von 1,5 weiter umgesetzt werden soll zu einem Polysaccharid-Kohlensäurederivat, ist es wenig sinnvoll, erheblich mehr als 5 Mol Phosgen pro Molgewichtseinheit der Methylcellulose einzusetzen.

Die Umsetzung mit Phosgen wird bei Temperaturen von 0 bis 80 °C, vorzugsweise 10 bis 70 °C, besonders bevorzugt 15 bis 60 °C durchgeführt. Phosgen wird mit einer solchen Geschwindigkeit in die Suspension flüssig, als Lösung oder gasförmig eindosiert, daß die freiwerdende Wärme kontrolliert abgeführt werden kann.

Die Reaktionsdauer hängt von der Art des Polysaccharids ab und von der gewählten Temperatur. Je besser die Polysaccharide aufgeschlossen sind, desto kürzer kann die Reaktionszeit sein. Um einen ausreichenden DS zu erzielen, sollte sie jedoch 3 h nicht unterschreiten. Längere Reaktionszeiten sind im Gegensatz zu den o.a. diskutierten Literaturangaben jedoch nicht schädlich und führen in der Regel zu einem weiteren Anstieg des DS. Für eine möglichst vollständige Nutzung des Acylierungsreagenzes empfiehlt sich also eine verlängerte Reaktionszeit, die natürlich zusammen mit anderen ökonomischen Fakten und einer gegebenenfalls möglichen Schädigung eines empfindlichen Polysaccharids betrachtet werden muß.

Die auf diese Weise mit Phosgen umgesetzten Polysaccharide enthalten Chlorkohlensäureester- und/oder Carbonatgruppen, erstere insbesondere dann, wenn ohne Base oder mit unzureichender Menge an Base gearbeitet wurde. Chlorkohlensäureestergruppen enthaltende Polysaccharide sind wenig lagerbeständig und werden zweckmäßig direkt weiter verarbeitet und z.B. mit Nucleophilen der Formel (I) umgesetzt. Carbonatgruppen enthaltende Polysaccharide können isoliert und gelagert werden. Auch sie sind natürlich der Umsetzung mit den o.a. Nucleophilen zugänglich und können in entsprechende Kohlensäurederivate umgewandelt werden.

Die Aufarbeitung der Polysaccharidcarbonate erfolgt nach an sich bekannten Methoden und richtet sich natürlich nach der Natur des umgesetzten Polysaccharids. Sie gestaltet sich besonders einfach, wenn man nach Abtrennen des flüssigen Teils des Reaktionsgemisches das Reaktionsprodukt durch Waschen mit geeigneten inerten Lösungsmitteln von den anhaftenden Resten des Reaktionsgemisches befreien kann. Dabei sollte weder Lösung noch Quellung erfolgen. Die geeigneten Lösungsmittel müssen von Fall zu Fall durch Vorversuche ermittelt werden. Vielfach sind die o.a. Lösungsmittel brauchbar. Häufig können auch Alkohole oder Wasser verwendet werden.

Die auf diesem Wege zugänglichen Kohlensäurederivate von Polysacchariden sind hervorragende Ausgangsprodukte zur Herstellung weiterer Derivate. Dazu werden sie umgesetzt mit protischen Nucleophilen oder deren Salzen der allgemeinen Formel (I)

R-X-M (I)

worin

- R $C_{1-18}$-Alkyl, bevorzugt $C_{1-12}$-Alkyl, besonders bevorzugt $C_{1-10}$-Alkyl, $C_{6-12}$-Aryl, bevorzugt $C_{6-10}$-Aryl, besonders bevorzugt $C_6$-Aryl, $C_{7-12}$-Aralkyl, bevorzugt $C_{7-10}$-Aralkyl, besonders bevorzugt $C_{7-8}$-Aralkyl

jeweils substituiert mit Olefin-, Alkin-, Amino-, Ether-, Thioether-, Carboxy-, Carbalkoxy-, Urethan-, quartären Ammoniumgruppen oder Halogenatomen, bevorzugt Olefin-, Amino-, Ether-, Carboxy-, Carbalkoxy-, quartären Ammoniumgruppen, besonders bevorzugt Olefin-, Amino-, Ether- und Carboxy-Gruppen.

- X O oder S, gegebenenfalls als Anion oder $NR^1$ mit $R^1$ H, $C_{1-6}$-Alkyl, Phenyl, $C_{7-8}$-Aralkyl, bevorzugt H, $C_{1-4}$-Alkyl, Phenyl, Benzyl, besonders bevorzugt H, Methyl, Ethyl,
- X bevorzugt O oder $NR^1$, besonders bevorzugt $NR^1$,
- M H, ein Alkalikation oder ein Ammoniumion, wenn X = $-O^{\ominus}$ oder $-S^{\ominus}$ darstellt, bevorzugt H oder Ammoniumion mit $-O^{\ominus}$.

Die auf dem erfindungsgemäßen Wege hergestellten Kohlensäurederivate von Polysacchariden können auch dienen zur Fixierung von biochemisch aktiven Verbindungen, wie beispielsweise von Enzymen oder Arzneimitteln, die langsam freigesetzt werden können.

Geeignete Nucleophile gemäß der obigen Formel (I) sind organische Hydroxyverbindungen wie Alkohole und Phenole, aliphatische und aromatische Mercaptane und aliphatische und aromatische Aminoverbindungen.

Geeignete Alkohole sind beispielsweise Methanol, Ethanol, Isopropanol, Butanol, Hexanol, Chlorethanol, Allylalkohol, Propinol, Methallylalkohol, N,N-Dimethylaminoethanol, Methoxyethanol, Ethoxyethanol, β-Hydroxyethyl-ethylsulfid, β-Hydroxy-ethylmethylsulfid, N-(β-Hydroxyethyl)-morpholin, N-Methyl-N'-(β-hydroxyethyl)-piperazin, Benzylalkohol, N,N-Dimethylaminopropanol.

Geeignete Phenole sind beispielsweise Phenol, Kresole, Xylenole, p-(N,N-Dimethylamino)-phenol, Salicylsäuremethylester, p-Hydroxybenzoesäureethylester, α-Naphthol, β-Naphthol.

Geeignete Mercaptane sind beispielsweise Benzylmercaptan, Phenylmercaptan, Allylmercaptan, Mercaptoethanol, Mercaptoessigsäure, Mercaptopropionsäure, Thioharnstoff, Mercaptobenzothiazol.

Geeignete Aminoverbindungen sind solche der Formel (II)

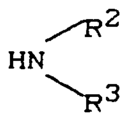

(II)

worin

$R^2$ und $R^3$ gleich oder verschieden sind und H, R mit der Maßgabe, daß nur einer der Reste Phenyl ist und zusätzlich zu R können $R^2$ und $R^3$ gemeinsam mit dem N-Atom und gegebenenfalls einem weiteren O- oder N-Atom einen 5- oder 6-gliedrigen Ring bilden.

Bevorzugt sind Amine der Formel (III)

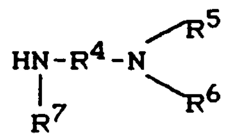

(III)

worin bedeuten

- $R^7$ H oder $C_1$-$C_4$-Alkyl,
- $R^4$ einen gegebenenfalls durch wenigstens ein O oder N-Atom unterbrochenen Alkylenrest,
- $R^5$ Wasserstoff, einen gegebenenfalls durch OH oder $OR^1$ substituierten $C_1$-$C_{18}$-Alkylrest, $C_5$-$C_6$-Cycloalkylrest, Benzyl oder (Meth)allyl,
- $R^6$ einen gegebenenfalls durch OH oder $OR^1$ substituierten $C_1$-$C_{18}$-Alkylrest, $C_5$-$C_6$-Cycloalkylrest oder Aryl,

wobei vorzugsweise nur jeweils einer der Reste

- $R^5$ bis $R^6$ Cycloalkyl oder $C_6$-$C_{18}$-Alkyl sein kann,

und wobei

- $R^7$ und $R^6$ zusammen mit $R^4$ und den beiden N-Atomen einen vorzugsweise sechsgliedrigen Ring und
- $R^5$ und $R^6$ zusammen mit dem N-Atom einen Ring bedeuten können, der vorzugsweise wenigstens ein weiteres Heteroatom, insbesondere Stickstoff und Sauerstoff, enthalten kann und der vorzugsweise 5- oder 6-gliedrig ist.

Geeignete Amine der Formel (II) sind beispielsweise Methylamin, Ethylamin, Propylamin, Butylamin, Isobutylamin, tert.-Butylamin, Pentylamin, Hexylamin, Cyclopentylamin, Pyrrolidin, Piperazin, N-Methylpiperazin, Morpholin, Cyclopentylamin, Cyclohexylamin, Octylamin, Decylamin, Isononylamin, Isooctylamin, Undecylamin, Dodecylamin, Stearylamin, Dimethylamin, Diethylamin, Dipropylamin, Methylbutylamin, Methylcyclohexylamin, Benzylamin, Methylbenzylamin, Ethanolamin, Isopropanolamin, Diethanolamin, Diisopropanolamin, Methylethanolamin, Ethylethanolamin, Methoxyethylamin, Glycin, Alanin, β-Alanin, ε-Aminocapronsäure oder die Ester der Aminosäuren wie Methyl-, Ethyl-, Benzylester oder oben genannten Aminosäuren, ferner Allylamin und Diallylamin.

Geeignete Amine der Formel (III) sind beispielsweise folgende:

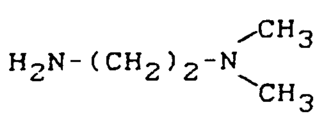 , 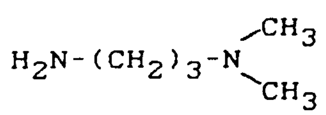 , 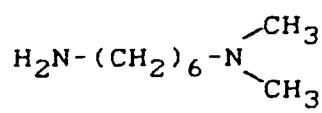 ,

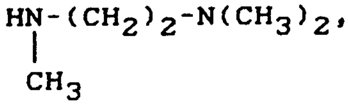, 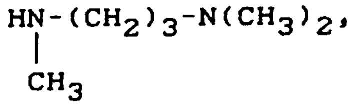,

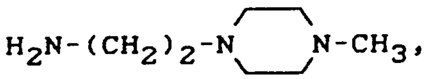, 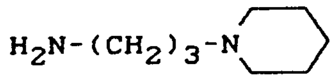,

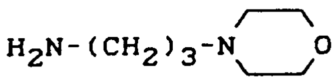, 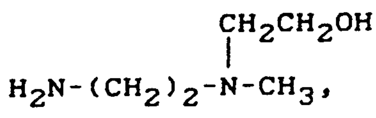,

$H_2N-(CH_2)_2-[NH-(CH_2)_2]_{1-5}-NH_2$, $H_2N-(CH_2)_3N(CH_2CH_2OH)_2$,

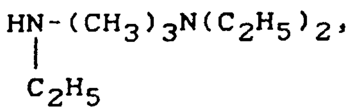,

$H_2N-(CH_2)_2-O-(CH_2)_2-N(CH_3)_2$,

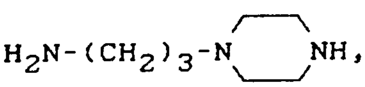, 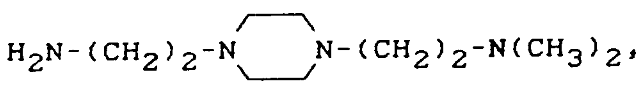,

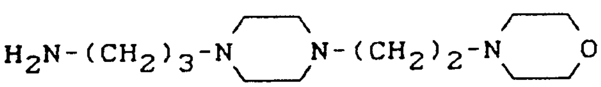, 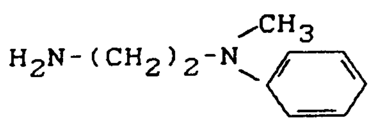,

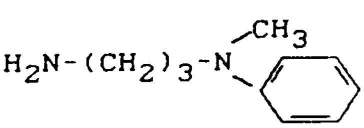, 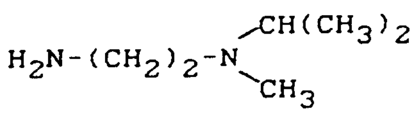,

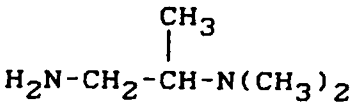, 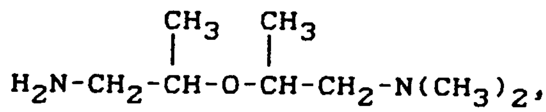,

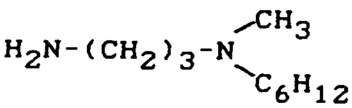, $H_2N-(CH_2)_3-N(CH_2CH_2OCH_3)_2$,

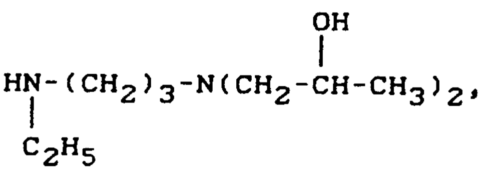, 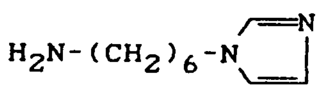,

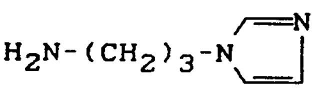, 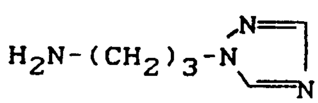,

bevorzugt sind beispielsweise:
$H_2N-(CH_2)_2N(CH_3)_2$, $H_2N-(CH_2)_3-N(CH_3)_2$, $H_2N-(CH_2)_6-N(CH_3)_2$, $H_2N-(CH_2)_3N(CH_2CH_2OH)_2$, $H_2N-(CH_2)_3-N(C_2H_5)_2$,

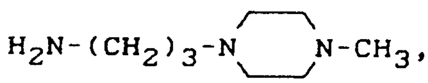,

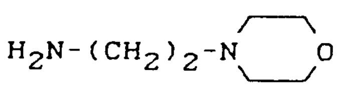, 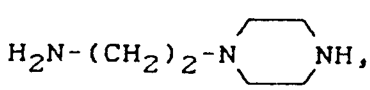,

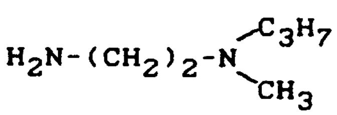, 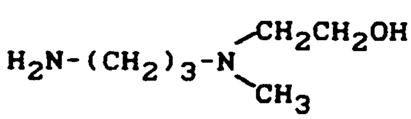,

$H_2N-(CH_2)_2-NH-(CH_2)_2-NH_2$

Die nach Umsetzung der phosgenierten Polysaccharide mit Aminen der Struktur III gewonnenen basischen Polysaccharide können mit Verbindungen der Formel (IV)

$R^8$ X (IV)

umgesetzt bzw. alkyliert werden, worin $R^8$ H, gegebenenfalls durch OH oder OR substituierter $C_{1-18}$-Alkylrest, $C_{5-6}$-Cycloalkylrest, Benzyl oder (Meth)allyl.

Man erhält auf diese Weise basische und kationische Polysaccharide, wie sie in der EP-A-367 003 beschrieben sind.

Geeignete Verbindungen IV sind beispielsweise: HCl, HBr, Methansulfonsäure, Toluolsulfonsäure,

Phosphorsäure, Benzolsulfonsäure, Methanphosphonsäure, Methylchlorid, Ethylchlorid, Propylchlorid, Butylchlorid, Chlorethanol, Chlorpropanol, Epichlorhydrin, 1-Chlorpropandiol-2,3, Chlorbutanol, 1-Chlor-2-methoxyethan, 1-Chlorpropanol-2, Benzylchlorid, (Meth)allylchlorid, Methylbromid, Methyliodid, Methylmethansulfonat, Methylbenzolsulfonat, Methyltoluolsulfonat, Trimethylphosphat, Methyl-O,O-dimethylphosphonat, Benzylmethansulfonat, Allyltosylat und Benzylmesylat.

Bevorzugt sind beispielsweise Methylchlorid, Chlorethanol, Dimethylsulfat, Methylmesylat, Methyltosylat, (Meth)allylchlorid, Benzylchlorid.

Die erfindungsgemäß erhältlichen basischen und kationischen Polysaccharide haben je nach Ausgangsprodukt und DS unterschiedliche Eigenschaften. Geht man von hochmolekularer Cellulose aus, so gewinnt man auch bei hohem DS wasserunlösliche Produkte, die für hochaktive Filtermaterialien geeignet sind. Nach einer vorausgehenden Alkalisierung und Vorquellung der Cellulose können bei entsprechend hohem Substitutionsgrad auch wasserlösliche Produkte gewonnen werden. Solche wasserlöslichen oder wasserquellbaren Derivate sind zugänglich aus weniger kristallinen Polysacchariden wie Dextrinen, Stärke, den Ethern der Cellulose, wie z.B. Methylcellulose.

Das erfindungsgemäße Verfahren soll anhand der folgenden Beispiele näher erläutert werden (%-Angaben sind - soweit nichts anderes angegeben - Gew.-%).

## Beispiele

### Beispiel 1

40 g (0,25 Mol) einer lufttrockenen handelsüblichen Fichtencellulose werden mit 2 g Wasser in 300 ml Methylenchlorid suspendiert und einige Stunden gerührt. Dann dosiert man in ca. 2 h bei 25 °C 99 g (1 Mol) Phosgen ein. Nach Rühren über Nacht wird das Lösungsmittel mit überschüssigem Phosgen im Vakuum abgezogen. Man erhält 121 g eines feuchten Rückstandes.

60,5 g davon werden in 150 ml Methylenchlorid suspendiert und bei Raumtemperatur mit 21 g 3-[N,N-Dimethylamino]-propylamin tropfenweise versetzt und 5 h bei Raumtemperatur gerührt, abgesaugt, mit Isopropanol mehrmals gewaschen und getrocknet: 23 g N-Gehalt 2,6 % Urethanbande im IR bei 1.730 $cm^{-1}$.

Die anderen 60,5 g werden ebenfalls in 150 ml Methylenchlorid suspendiert mit 47 g (0,5 Mol) Phenol versetzt und 50 g (0,5 Mol) Triethylamin zugetropft. Nach 2,5 h Rühren wird abgesaugt, mit Isopropanol gewaschen und getrocknet.
18 g starke Carbonatbande im IR bei 1.770 $cm^{-1}$.

### Beispiel 2

40 g (0,25 Mol) einer Fichtencellulose (wie in Beispiel 1), 156 g (2,0 Mol) Pyridin (über KOH getrocknet), 750 ml Methylenchlorid und 1,5 g Wasser werden über Nacht bei Raumtemperatur gerührt und dann werden 99 g (1,0 Mol) Phosgen bei 20 °C in die Suspension in 1 h eindosiert. Nach weiteren 8 h Rühren bei Raumtemperatur wird das Lösungsmittel im Vakuum abgezogen, der Rückstand wieder in Methylenchlorid aufgenommen, die Suspension bei 25 °C mit 102 g (1 Mol) 3-(N,N-Dimethylamin)-propylamin tropfenweise versetzt und noch 4 h bei Raumtemperatur gerührt. Nach Absaugen und mehrmaligem Waschen mit Methylenchlorid und Isopropanol und Trocknen bei 50 °C im Vakuum erhält man 104 g Produkt mit einem N-Gehalt von 10,0 % entsprechend einem Substitutionsgrad von 1.

### Beispiel 3

45 g (0,25 Mol) einer Methylcellulose mit einem DS von 1,4 werden mit 2 g Wasser in 400 ml Methylenchlorid suspendiert und nach einigen Stunden Rühren mit 99 g (1 Mol) Phosgen umgesetzt. Nach Rühren über Nacht bei Raumtemperatur werden Lösungsmittel und überschüssiges Phosgen im Vakuum abgezogen. Ein kleiner Teil des Rückstandes wird abgetrennt, mehrmals mit Methylenchlorid gewaschen, rasch getrocknet im Vakuum und analysiert: IR-Bande bei 1.784 $cm^{-1}$, 6,5 % $CO_2$-Gehalt, bestimmt durch Verseifung.

Der Rückstand wird wieder in Methylenchlorid aufgenommen, mit 76 g (0,75 Mol) 3-(N,N-Dimethylamino)-propylamin tropfenweise versetzt, 5 h bei Raumtemperatur gerührt, über Nacht stehen gelassen, und wie in Beispiel 2 aufgearbeitet. Man erhält 62 g mit einem N-Gehalt von 5,2 % und einer IR-Bande bei 1.730 $cm^{-1}$.

Beispiel 4

53 g (0,25 Mol) Hydroxyethylcellulose mit einem DS von 1,1 und einem Wassergehalt von 6,5 Gew.-% wird mit 79 g (1 Mol) Pyridin in 700 ml Methylenchlorid einige Stunden gerührt. Dazu werden in 1 h 99 g (1 Mol) Phosgen dosiert und weitere 5 h bei Raumtemperatur gerührt. Dann zieht man im Vakuum Methylenchlorid ab, nimmt den Rückstand in Toluol auf und tropft 101 g (1 Mol) 3-(N,N-Dimethylamino)-propylamin bei Raumtemperatur in 2 h zu, erhitzt auf 80 ° C, läßt noch 4 h bei 80 ° C rühren, saugt ab, wäscht mehrmals mit Isopropanol, trocknet im Vakuum bei 60 ° C und erhält dann 133 g Cellulosederivat mit einem N-Gehalt von 10,3 % und einem Chlorgehalt von 18 %, entsprechend einem Substitutionsgrad von 1 bezüglich der Urethanfunktion.

**Patentansprüche**

**1.** Verfahren zur Herstellung von Kohlensäurederivaten von Polysacchariden durch Umsetzung mit reaktiven Kohlensäurederivaten, dadurch gekennzeichnet, daß man Polysaccharide zunächst mit Phosgen und gegebenenfalls weiter mit protischen nucleophilen Substanzen und/oder deren Salzen der allgemeinen Formel (I)

R-X-M (I)

umsetzt, worin

R $C_{1-18}$-Alkyl, $C_{6-12}$-Aryl, $C_{7-12}$-Aralkyl, jeweils substituiert mit Olefin-, Alkin-, Amino-, Ether-, Thioether-, Carboxy-, Carbalkoxy-, Urethan-, quartären Ammoniumgruppen oder Halogenatomen,

X O oder S, gegebenenfalls als Anion oder $NR^1$ mit $R^1$ H, $C_{1-6}$-Alkyl, Phenyl, $C_{7-8}$-Aralkyl und

M H, ein Alkalikation oder ein Ammoniumion, wenn X = $O^{\ominus}$ oder $S^{\ominus}$ darstellt.